# EUROPEAN PATENT APPLICATION

(11) **EP 4 674 365 A1**
(43) Date of publication of application: **07.01.2026**
(21) Application number: 24763890.1
(22) Date of filing: 27.02.2024
(51) Int. Cl.: A61B 17/88, A61L 27/16, A61L 27/18

(54) **INJECTION DEVICE, AND BIOMATERIAL**

(30) Priority: 01.03.2023 JP 2023031368
(71) Applicant: The University of Osaka, Osaka 565-0871 (JP)
(72) Inventor: UKON, Yuichiro, Suita-shi, Osaka 565-0871 (JP); MASUDA, Hirotada, Suita-shi, Osaka 565-0871 (JP); MAE, Hirokazu, Suita-shi, Osaka 565-0871 (JP); YAGI, Masakazu, Suita-shi, Osaka 565-0871 (JP); SAWA, Yoshiki, Suita-shi, Osaka 565-0871 (JP); OKA, Yuma, Kobe-shi, Hyogo 651-0073 (JP); YAMAWAKI, Koya, Kobe-shi, Hyogo 651-0073 (JP); MATSUMOTO, Kazuya, Kobe-shi, Hyogo 651-0073 (JP)
(74) Representative: KATZAROV S.A.
(86) International application number: PCT/JP2024/006975
(87) International publication number: WO 2024/181411

(57) **Abstract**

An injection instrument (1) includes: an injector (20) that is a cylindrical body having one or more holes, wherein the injector receives a flow of a mixed solution, which is a mixture of a first solution and a second solution that hardens by being mixed with the first solution, in a state in which the injector is inserted in an opening formed in a bone of a target, and the injector causes the mixed solution received to flow out of the injector (20) from the one or more holes to permeate inside of the bone and harden.

## Description

### [Technical Field]

The present invention relates to an injection instrument and a biomaterial.

### [Background Art]

A reduction in the bone mineral density of the femur caused by, for example, osteoporosis can result in a femur fracture. A patient with a fractured femur has a decreased standard of living. A fractured femur is treated by, for example, open reduction and internal fixation or bipolar hip arthroplasty.

There is known a method of treating a fracture by repairing the bone using a curable polymer (see, for example, Patent Literature (PTL) 1).

### [Citation List]

### [Patent Literature]

[PTL 1] US Patent No. 8329204

### [Non Patent Literature]

[NPTL 1] NORITAKE CO., LIMITED, "What's a Static Mixer?", [online], [retrieved on February 24, 2023], Internet <URL: https://www. noritake. co.jp/products/support/detail/13/>

### [Summary of Invention]

### [Technical Problem]

It is conceived that measures are provided to a patient having experience of a femur fracture (first fracture) to prevent a fracture in the other femur. There has been no known appropriate instrument capable of preventing a patient with experience of the first fracture from having a fracture in the other femur.

The present invention aims to provide an injection instrument and so forth capable of increasing the strength of a bone.

### [Solution to Problem]

To solve the foregoing problem, the injection instrument according to an aspect of the present invention is an injection instrument including: an injector that is a cylindrical body having one or more holes, wherein the injector receives a flow of a mixed solution in a state in which the injector is inserted in an opening formed in a bone of a target, the mixed solution being a mixture of a first solution and a second solution that hardens by being mixed with the first solution, and the injector causes the mixed solution received to flow out of the injector from the one or more holes to permeate inside of the bone and harden.

### [Advantageous Effects of Invention]

The injection instrument of the present invention is capable of increasing the strength of a bone.

### [Brief Description of Drawings]

[FIG. 1]
   An explanatory diagram showing an example of the external view of an injection instrument according to an embodiment.
[FIG. 2]
   An explanatory diagram showing an example of the external view of an injector according to the embodiment.
[FIG. 3]
   An explanatory diagram showing an example of the usage state of the injection instrument according to the embodiment.
[FIG. 4]
   A flow diagram showing a method of using the injection instrument according to the embodiment.
[FIG. 5]
   An explanatory diagram showing an example of a bone before subjected to a procedure performed by the injection instrument according to the embodiment.
[FIG. 6]
   An explanatory diagram showing a first example of the bone after subjected to the procedure performed by the injection instrument according to the embodiment.
[FIG. 7]
   An explanatory diagram showing a second example of the bone after subjected to the procedure performed by the injection instrument according to the embodiment.

### [Description of Embodiment]

### (Underlying Knowledge Forming Basis of the Present Invention)

The inventors have found that problems described below arise in the technique related to the prevention of fractures described in the "Background Art" section.

It has been said that a reduction in the bone mineral density of the femur caused by, for example, osteoporosis advances after the age of 60. Approximately 40% of the patients who has had a fractured femur have difficulty in walking, resulting in a decrease in their standard of living. A fractured femur is treated by, for example, open reduction and internal fixation or bipolar hip arthroplasty.

It is said that patients with experience of a fracture in one femur have a relatively high risk of having a fracture in the other (contralateral) femur (contralateral fracture). It becomes more difficult or even impossible for patients with experience of a contralateral fracture to walk, resulting in a further decrease in their standard of living.

In some cases, drug treatment (injection treatment or oral treatment) is provided to patients with experience of a fracture in one femur. Under the present circumstances, however, the prevention rate of contralateral fractures by means of drug treatment is said to be 30% to 50%, which is not very high.

It is conceived that measures are provided to a patient with experience of a femur fracture (first fracture) to prevent a contralateral fracture. There has been no known appropriate instrument capable of preventing a patient with experience of the first fracture from having a contralateral fracture.

The present invention provides an injection instrument capable of increasing the strength of a bone.

The following illustrates the invention obtained from the details disclosed by this DESCRIPTION, and effects and so forth obtained from the invention.

(1) An injection instrument including: an injector that is a cylindrical body having one or more holes, wherein the injector receives a flow of a mixed solution in a state in which the injector is inserted in an opening formed in a bone of a target, the mixed solution being a mixture of a first solution and a second solution that hardens by being mixed with the first solution, and the injector causes the mixed solution received to flow out of the injector from the one or more holes to permeate inside of the bone and harden.

According to the above aspect, the injection instrument enables the mixed solution to permeate inside of the bone from the opening formed in the bone, and then harden. This enables the mixed solution that has hardened inside of the bone to serve as a relatively high-strength biomaterial inside of the bone. As a result, the strength of the bone of the target is increased. As described above, the injection instrument is capable of increasing the strength of the bone.

(2) The injection instrument according to (1), wherein the one or more holes include: one or more side holes located at one or more positions in a circumferential direction of the cylindrical body; or one hole located at, among one end of the cylindrical body into which the mixed solution flows and an other end different from the one end, the other end.

According to the above aspect, by causing the mixed solution to flow from the injection instrument using, as an outlet from which the mixed solution flows out, the one or more side holes of the cylindrical body or the hole located at the other end of the cylindrical body, it is possible to more easily enable the mixed solution to permeate inside of the bone. As described above, the injection instrument is capable of more easily increasing the strength of the bone.

(3) The injection instrument according to (1), wherein the one or more holes are a plurality of side holes located at a plurality of positions in an axial direction of the cylindrical body, and a diameter of each of the plurality of side holes increases with increasing distance of the side hole from one end of two ends of the cylindrical body, the one end being an end into which the mixed solution flows.

According to the above aspect, in the injection instrument, the diameter of each of the plurality of side holes increases with increasing distance of the side hole from the one end from which the mixed solution flows. This enables the mixed solution to flow from the plurality of side holes relatively evenly and to permeate evenly around the holes. An external force applied to the mixed solution that flows into the injector decreases with increasing distance of the external force from the one end from which the mixed solution flows. This is why the mixed solution evenly flows from the plurality of side holes by causing the mixed solution to flow out under the application of the force that decreases with increasing distance of the force from the one end. As described above, the injection instrument is capable of more evenly increasing the strength of the bone around the target portion.

(4) The injection instrument according to (3), wherein at least one of the plurality of side holes is oval in shape.

According to the above aspect, the injection instrument enables the mixed solution to flow from the side holes in an oval shape. The holes in an oval shape make it possible for the injection instrument to more easily cause the mixed solution to flow out. With this, it is possible for the injection instrument to increase the strength of the bone by more easily enabling the mixed solution to flow out.

(5) The injection instrument according to any one of (1) to (4), wherein the opening is an opening formed by inserting the injector into the bone.

According to the above aspect, the insertion of the injector forms an opening in the target portion, bringing the injector in a state of being inserted in the opening. This eliminates the necessity of using a drilling instrument other than the injection instrument, thus achieving the advantage of simplifying the usage procedure of the injection instrument. With this, it is possible for the injection instrument to more easily increase the strength of the bone.

(6) The injection instrument according to any one of (1) to (4), wherein the opening is an opening formed by a drilling instrument different from the injector.

According to the above aspect, an opening is easily formed using a drilling instrument suitable for forming an opening. With this, it is possible for the injection instrument to more easily increase the strength of the bone.

(7) The injection instrument according to any one of (1) to (4), further including: a mixer connected to one end of two ends of the injector, the one end being an end into which the mixed solution flows, wherein the mixer receives the first solution and the second solution, and causes the mixed solution of the first solution and the second solution received to flow into the injector.

According to the above aspect, in the injection instrument, the mixer mixes the solutions received (i.e., the first solution and the second solution), after which the injector causes the mixed solution to flow from the opening. It is thus possible for the injection instrument to increase the strength of the bone using the first solution and the second solution received, without the necessity of, for example, a process of mixing the first solution and the second solution in advance. As described above, the injection instrument is capable of more easily increasing the strength of the bone.

(8) The injection instrument according to any one of (1) to (4), wherein the target has a fracture in one femur, and the bone is an other femur of the target.

According to the above aspect, the injection instrument contributes to reducing the occurrence rate of a fracture in the other femur (i.e., contralateral fracture). This consequently contributes to preventing a decrease in the standard of living of patients with a contralateral fracture caused by difficulty or inability to walk. As described above, the injection instrument contributes to preventing a decrease in the standard of living of patients with a contralateral fracture by increasing the strength of their bones.

(9) The injection instrument according to any one of (1) to (4), wherein the bone is one of the following bones of the target diagnosed with osteoporosis: a femur; a vertebral body; a distal radius; and a proximal humerus.

According to the above aspect, the injection instrument contributes to decreasing the occurrence rate of a fracture in the femur, the vertebral body, a distal radius, or a proximal humerus of the target diagnosed with osteoporosis. This contributes to preventing a decrease in the standard of living of the target caused by a fracture. As described above, the injection instrument contributes to preventing a decrease in the standard of living of the target diagnosed with osteoporosis by increasing the strength of the bone.

(10) The injection instrument according to any one of (1) to (4), wherein the injector receives the flow of the mixed solution that has pregelatinized, and the injector causes the mixed solution that has pregelatinized to flow out of the injector to permeate inside of the bone, gelatinize, and then harden.

According to the above aspect, since the mixed solution is in pregelatinized form, it is possible for the injection instrument to prevent the mixed solution from excessively permeate and spread inside of the bone and from flowing into vessels. With this, it is possible for the injection instrument to enable the mixed solution to remain in the target portion and appropriately harden the target portion. It is thus possible for the injection instrument to more appropriately increase the strength of the bone.

(11) A biomaterial including: an injectable gel; and a polymer of polymethyl methacrylate (PMMA) and a cross-linking agent.

According to the above aspect, it can be considered that the biomaterial, which has become a polymer like a double network gel (link) by the polymerization with MMA inside the injectable gel, has hard and viscous physical properties, that is, physical properties suitable for replacing the bone function. MMA is said to be biotoxic in monomer form, but is considered safe when it turns into a polymer. It can be considered that, by the injectable gel retaining MMA, the above biomaterial will not leak until MMA forms a polymer, achieving high safety. As described above, the above biomaterial is capable of increasing the strength of the bone.

(12) The biomaterial according to claim (11), wherein the injectable gel is a Tetra-Polyethyleneglycol (PEG) gel.

According to the above aspect, it can be considered that the biomaterial, which has become a polymer like a double network gel (link) by the polymerization with MMA inside the Tetra-PEG gel serving as the injectable gel, has hard and viscous physical properties, that is, physical properties suitable for replacing the bone function. It can be considered that, by the Tetra-PEG gel serving as the injectable gel retaining MMA, the above biomaterial will not leak until MMA forms a polymer, achieving much higher safety. As described above, the above biomaterial is capable of increasing the strength of the bone.

Hereinafter, a certain exemplary embodiment is described in greater detail with reference to the accompanying Drawings.

The exemplary embodiment described below shows a preferred embodiment of the present invention. The numerical values, shapes, materials, elements, the arrangement and connection of the elements, steps, the processing order of the steps etc. shown in the following exemplary embodiment are mere examples, and therefore do not limit the scope of the present invention. Therefore, among the elements in the following exemplary embodiment, those not recited in any one of the independent claims that show the broadest concepts of the present invention are described as optional elements that form a more preferred embodiment. Note that the same reference marks are assigned to the same elements, and their descriptions can be omitted.

### [Embodiment]

The present embodiment describes an injection instrument capable of increasing the strength of a bone. The injection instrument is capable of increasing the strength of a bone of an animal, and more specifically the strength of a bone of a person.

The following describes an example case where the bone to be strengthened by the injection instrument is the femur of a person, but the present invention is not limited to this example. When the bone to be strengthened by the injection instrument is the other femur of a target having had a fracture in one femur, the present invention contributes to reducing the occurrence rate of a contralateral fracture. This consequently contributes to avoiding a decrease in the standard of living of patients with a contralateral fracture caused by difficulty or inability to walk.

Note that a person who uses injection instrument 1 is referred to as the "user". The user is, for example, a physician.

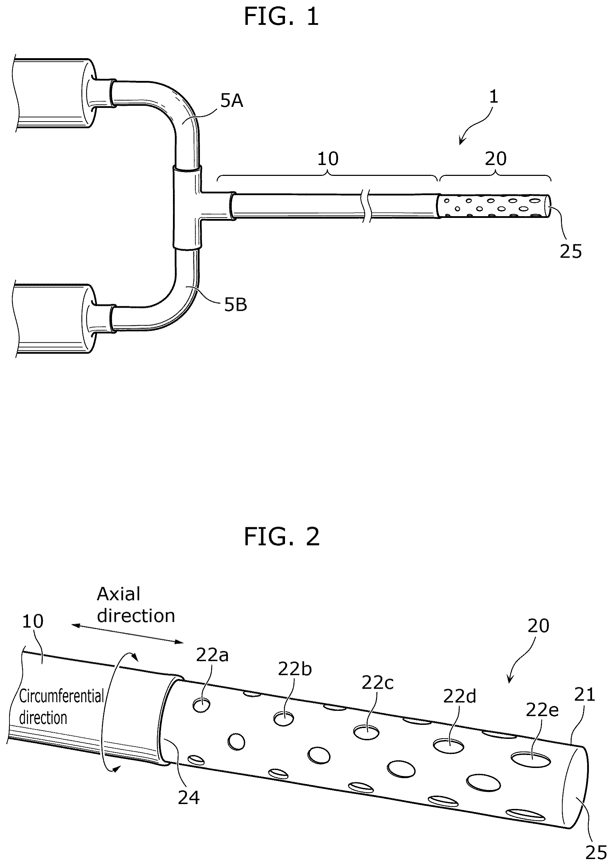

FIG. 1 is an explanatory diagram showing an example of the external view of injection instrument 1 according to the present embodiment. Injection instrument 1 increases the strength of a portion in the femur (also referred to as "target portion") by enabling a gel to permeate the target portion and harden.

As shown in FIG. 1, injection instrument 1 includes inlets 5A and 5B, mixer 10, and injector 20.

Each of inlets 5A and 5B is an inlet into which a solution flows. A solution that flows into inlet 5A (also referred to as "solution A" or "first solution") and a solution that flows into inlet 5B (also referred to as "solution B" or "second solution") flow into mixer 10. Solution A and solution B flow into inlets 5A and 5B under the application of an external force.

Solution A and solution B have the property of hardening when mixed with each other. Solution A or solution B may include insoluble particulates (solids).

More specifically, solution A and solution B have the property of gelatinizing by being mixed with each other and then hardening. The gel produced by the mixture of solution A and solution B can be an injectable gel. The injectable gel is injected to inside the body in liquid form and then gelatinizes inside the body after being injected.

Examples of the gelation reaction may be any one of, but not limited to one of, radical polymerization, condensation polymerization, polymer flocculation accompanied by an environmental change, physical bonding of polymer chains, and chemical bonding of polymer chains. A more specific gelation reaction that can be used is radical polymerization between monomer units including polyethylene glycol, terminal cross-linking reaction of branched polyethylene glycol, gelation reaction of Pluronic (registered trademark) F-127 by micellar aggregation caused by a temperature change, or cross-linking reaction of hyaluronic acid and polyglutamic acid by enzyme catalyst.

The gel produced by the mixture of solution A and solution B is, for example, a high-strength gel, which can be more specifically a Tetra-Polyethyleneglycol (PEG) gel, a nanocomposite gel, a double network gel, or a side-ring gel. In general, these gels using PEG as a polymer chain are known to be highly biocompatible, and serve as relatively high-strength biomaterials when they harden. Solution A is, for example, a methyl methacrylate (MMA) solution (including a cross-linking agent and am accelerator) in which pre-Tetra-PEG gel A is dissolved. Solution B is, for example, an MMA solution (including an initiator) in which pre-Tetra-PEG gel B is dissolved, and includes an artificial bone material (e.g., hydroxyapatite (HA)). In this case, solution A and solution B are mixed to form a Tetra-PEG gel, in which MMA is polymerized. In a predetermined time (e.g., on the order of 30 seconds to 5 minutes) after solution A and solution B are mixed, the mixed solution gelatinizes and then hardens. As described above, a biomaterial including a Tetra-PEG gel and a polymer of polymethyl methacrylate (PMMA) and a cross-linking agent is produced from solution A and solution B. Such biomaterial produced can contribute to increasing the strength of the bone.

There is no known biomaterial for bone strengthening that includes a Tetra-PEG gel and PMMA. A Tetra-PEG gel, by itself, has the property of being low in strength as a material for bone strengthening. PMMA, by itself, has already been clinically applied as a material for bone cement used for fracture treatment or other purposes, but is said to cause atypical fractures because of its excessive hardness.

Mixer 10 mixes solution A and solution B. More specifically, mixer 10 includes a tube that serves as a flow path through which solution A that has flowed into inlet 5A and solution B that has flowed into inlet 5B are mixed while flowing. Mixer 10 has, for example, a static mixer structure (see NPTL 1). The number of elements in the static mixer is 5 to 24, and preferably on the order of 10 to 20.

The viscosity of the mixed solution produced by the mixture of solution A and solution B gradually increases as the gelatinization gradually progresses, making it harder for the mixed solution to flow through the flow path. The mixed solution flows from inlets 5A and 5B to mixer 10, and flows through the flow path from mixer 10 to injector 20 under the application of force in the direction from mixer 10 to injector 20. The external force applied to solution A and solution B that flow into inlets 5A and 5B serves as the force that causes the mixed solution to flow through the flow path. Mixer 10 causes the mixed solution produced by the mixture of solution A and solution B to flow from the flow path to injector 20.

The material of the tube is, for example, natural rubber, silicone rubber, perfluoroelastomer (FFKM), or fluororesin (PTFE, ETFE, PFA, etc.). The length of the tube is, for example, on the order of 10 cm to 20 cm. The diameter of the tube may be constant (e.g., on the order of 5 mm) regardless of the position in the tube or may vary depending on the position in the tube.

Injector 20 causes the mixed solution produced by the mixture of solution A and solution B to be injected into the femur of the target. Injector 20 is a cylindrical body having one or more holes. The material of injector 20 is, for example, stainless steel.

The mixed solution produced by the mixture of solution A and solution B flows from mixer 10, with injector 20 inserted in an opening formed in the target portion in the bone. The mixed solution that flows into injector 20 is a pregelatinized mixed solution (stated differently, a mixed solution in which a cross-linking reaction or a polymerization reaction, for example, has partially progressed). Injector 20 causes the mixed solution that has flowed thereto to flow out of injector 20 from one or more holes. The mixed solution that has flowed out permeates inside of the bone and hardens. The hardening of the mixed solution is the hardening resulting from the mixture of solution A and solution B.

Stated differently, injector 20 causes the mixed solution to flow out of injector 20 from the one or more holes, thereby enabling the mixed solution that has flowed out to permeate inside of the bone and harden. More specifically, injector 20 causes the pregelatinized mixed solution to flow out of injector 20, thereby enabling the mixed solution that has flowed out to permeate inside of the bone to gelatinize, and then harden.

The mixed solution that has hardened inside of the bone serves as a replacement of the function of the cancellous bone inside of the bone, thereby contributing to increasing the strength of the bone. By maintaining its shape when an external force is applied to the bone, such mixed solution contributes to preventing the occurrence of a fracture or other anomalies.

Here, the one or more holes include one or more side holes located at one or more positions in the circumferential direction of the cylindrical body or a hole located at end portion 25. Stated differently, injector 20 may have one or more side holes without having a hole at end portion 25. Injector 20 may also have a hole at end portion 25 without having side holes. Alternatively, injector 20 may have one or more side holes, and a hole at end portion 25.

The opening formed in the target portion in the bone may be an opening formed by the insertion of injector 20 into the bone. In this case, injector 20 is required to have a structure that enables a tip (stated differently, end portion 25) of injector 20 to be inserted into the bone (e.g., injector 20 has a pointed tip).

The opening formed in the target portion in the bone may also be an opening formed by a drilling instrument that is different from injector 20. The drilling instrument can be a drill or a needle. In this case, the opening is formed by the drilling instrument in advance and then injector 20 is inserted into such formed opening.

The following describes injector 20 in detail.

FIG. 2 is an explanatory diagram showing an example of the external view of injector 20 according to the present embodiment.

Injector 20 shown in FIG. 2 includes side wall 21 and a plurality of side holes 22a, 22b, 22c, 22d, 22e, etc. (also referred to as "side holes such as side hole 22a"). Side holes such as side hole 22a correspond to the plurality of side holes located at the plurality of positions in the axial direction of injector 20. Of the two ends of injector 20 shown in FIG. 2, end portion 24 is connected to mixer 10 and end portion 25 is closed.

The internal portion of side wall 21 forms a flow path through which the mixed solution that has flowed from mixer 10 flows.

Side hole 22a is a side hole formed in side wall 21. Side hole 22a serves as an outlet from which the mixed solution that flows through the flow path inside side wall 21 flows out of injector 20. The mixed solution is pushed by the external force applied to solution A and solution B that flow into inlets 5A and 5B to flow out of injector 20 from side hole 22a.

Each of side holes 22b, 22c, 22d, 22e, etc. is the same side hole as side hole 22a.

Side holes such as side hole 22a may be in any shapes, and can be, for example, a circular, an oval, or a polygonal in shape. Side holes such as side hole 22a in a circular shape or an oval shape achieve an advantage of not obstructing the flowing of the pregelatinized mixed solution to outside. At least one of side holes such as side hole 22a may be in an oval shape, or all of side holes such as side hole 22a may be in an oval shape.

Side holes such as side hole 22a may have approximately the same diameters or different diameters (widths when having a polygonal shape, which is applicable to the description that follows). For example, the diameter of each of side holes such as side hole 22a may increase with increasing distance of the side hole from end portion 24. The force applied to the mixed solution inside injector 20 is the external force applied to solution A and solution B that flow into inlets 5A and 5B, and thus decreases with increasing distance of the force from end portion 24. In view of this, by structuring each of side holes such as side hole 22a to increase in diameter with increasing distance of the side hole from end portion 24, it is possible to contribute to enabling the mixed solution to evenly permeate the target portion.

Side holes such as side hole 22a may have random diameters, regardless of the positions in injector 20.

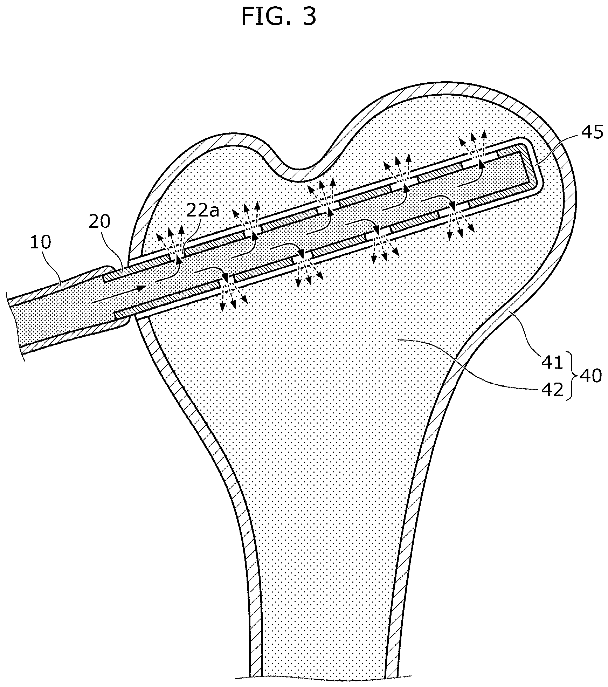

FIG. 3 is an explanatory diagram showing an example of the usage state of injection instrument 1 according to the present embodiment.

FIG. 3 shows a cross section of femur 40. Opening 45 is formed in femur 40 shown in FIG. 3, and injector 20 is in a state of being inserted in opening 45.

Femur 40 includes cortical bone 41 and cancellous bone 42. Cortical bone 41 is a bone substance located on the outer side of the bone and is a bone tissue having a relatively high density. Cancellous bone 42 is a bone substance located on the inner side of the bone and is a bone tissue having a relatively low density. Cancellous bone 42 is a porous tissue formed of bone beams arranged in a mesh pattern.

Opening 45 is formed to penetrate through cortical bone 41 and cancellous bone 42. Opening 45 may be an opening formed by the insertion of injector 20 into the bone or an opening formed by a drilling instrument, as described above.

When the mixed solution flows out from side holes such as side hole 22a of injector 20 with injector 20 inserted in opening 45, such mixed solution that has flowed out permeates inside of cancellous bone 42 and remains inside cancellous bone 42. The depth to which the mixed solution permeates inside of cancellous bone 42 is, for example, on the order of 3 to 10 mm. The reason that the mixed solution permeates only as far as a distance of 3 to 10 mm around opening 45 inside cancellous bone 42 is because the viscosity of the mixed solution is increasing due to the gelatinization of the mixed solution in progress. The permeation of the mixed solution into cancellous bone 42 can be based on capillary action. The mixed solution that remains inside of cancellous bone 42 hardens after a predetermined time has elapsed after the mixture of solution A and B.

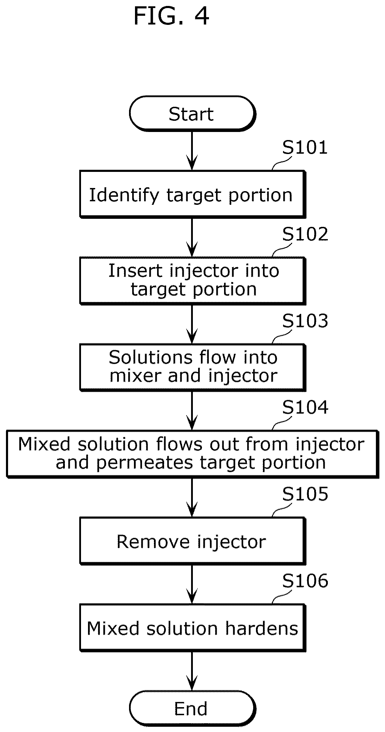

FIG. 4 is a flow diagram showing a method of using injection instrument 1 according to the present embodiment. The present description shows an example case where opening 45 is an opening formed by the insertion of injector 20 into the bone.

In step S101, the user identifies the target portion in the bone to be strengthened by injection instrument 1. The user identifies the target portion by, for example, identifying a portion in the femur where the strength should be increased or a portion in the femur where the bone mineral density has decreased, through the observation of the bone mineral density of the femur by means of X-ray fluoroscopy.

In step S102, the user inserts injector 20 into the target portion. The insertion of injector 20 forms opening 45 in the target portion, bringing injector 20 in a state of being inserted in opening 45.

In step S103, mixer 10 receives a flow of solutions (i.e., solution A and solution B), and causes the mixed solution produced by the mixture of such solutions to flow into injector 20.

In step S104, the mixed solution flows from injector 20 into opening 45, and the mixed solution that has flowed out permeates the target portion. The mixed solution that has flowed out gelatinizes while permeating the target portion.

In step S105, the user removes injector 20.

In step S106, the mixed solution that has permeated the target portion hardens.

Through a series of steps in the usage method shown in FIG. 4, the gel permeates and hardens inside of the target portion, thereby increasing the strength of the target portion in the femur.

The following describes an example of the bone before and after subjected to a procedure performed by injection instrument 1.

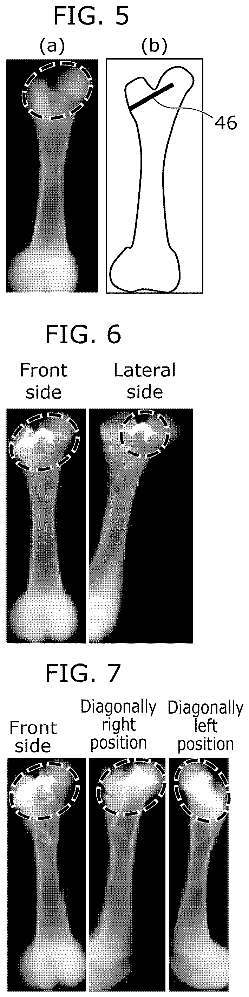

FIG. 5, FIG. 6, and FIG. 7 are explanatory diagrams showing examples of the bone before and after subjected to the procedure performed by injection instrument 1 according to the present embodiment. FIG. 5, FIG. 6, and FIG. 7 show images of the target bone that are captured using X-rays. In such images, darker white represents higher density. Here, a PEG solution that mimics the mixed solution produced by the mixture of solution A and solution B is used.

(a) in FIG. 5 shows an example of the bone before subjected to the procedure performed by injection instrument 1. FIG. 5 shows the target portion surrounded by a dashed frame. The orientation shown in FIG. 5 is assumed to be frontal.

Also, (b) in FIG. 5 shows graphics 46 representing the position of opening 45 to be formed in the bone shown in (a) in FIG. 5. Opening 45 has not yet been drilled in the bone shown in (a) in FIG. 5.

FIG. 6 is an explanatory diagram showing a first example of the bone after subjected to the procedure performed by injection instrument 1 according to the present embodiment. FIG. 6 shows a state in which 5 ml of the solution is injected into the target portion by injection instrument 1.

In FIG. 6, the target portion surrounded by the dashed frame is represented as darker white than in (a) in FIG. 5. This indicates that the solution that has flowed out of injector 20 into opening 45 is permeating inside of the bone (i.e., inside of the cancellous bone).

FIG. 7 is an explanatory diagram showing a second example of the bone after subjected to the procedure performed by injection instrument 1 according to the present embodiment. FIG. 7 shows a state in which 5 ml of the solution is further injected into the target portion by injection instrument 1 from the state shown in FIG. 6.

In FIG. 7, the target portion surrounded by the dashed frame is represented as much darker white, spreading over a wider area than in FIG. 6. This indicates that the gel that has flowed out of injector 20 into opening 45 is permeating a wider area inside of the bone (i.e., inside of the cancellous bone).

As described above, it has been verified that injection instrument 1 enables the gel to permeate a relatively wide area in the target portion.

Compared with conventional treatment techniques, the technique of increasing the strength of the bone using injection instrument 1 has the characteristics described below. The conventional treatment techniques include, for example, a method of using drugs for osteoporosis and Localized Bone Enhancement Procedures (LOEP).

### (1) Less invasive

The technique of increasing the strength of the bone using injection instrument 1 requires drilling for the formation of opening 45, but the area for the drilling is relatively small.

In contrast, LOEP involves a relatively large area for drilling for implantation.

As described above, the technique of increasing the strength of the bone using injection instrument 1 is less invasive because the area required for drilling is relatively small.

### (2) Shorter surgical time and fewer treatments

The surgical time required by the technique of increasing the strength of the bone using injection instrument 1 is on the order of 20 minutes. The number of treatments required by the technique of increasing the strength of the bone using injection instrument 1 is once. When the bone to be strengthened by injection instrument 1 is one femur of the target who has had a fracture in the one femur, measures for increasing the strength of the contralateral femur (i.e., the unfractured femur) are provided under anesthesia during the surgery for treating the fracture is ongoing. This contributes to reducing the occurrence rate of a contralateral fracture.

In contrast, the time required by LOEP is on the order of 75 minutes or longer. The time required for a surgery for treating a fractured femur using an implant, such as an intramedullary nail, a Hanson pin, or an artificial femoral head, is on the order of 30 to 60 minutes.

The method of using drugs for osteoporosis does not require a surgical operation, but requires the target to take drugs for a plurality of times.

As described above, the technique of increasing the strength of the bone using injection instrument 1 is more convenient due to a relatively short surgical time and a relatively small number of treatments required.

### (3) Higher safety

In the technique of increasing the strength of the bone using injection instrument 1, a high-strength gel that is known to be highly biocompatible can be used.

In contrast, drugs used in the method of using drugs for osteoporosis are known to have side effects such as jaw osteonecrosis and the development of carcinogenicity or an atypical fracture. In addition, LOEP is known to cause massive bleeding accompanied by bone curettage and perioperative complications caused by hemodynamic change, due to which the safety is relatively low.

As described above, the safety of the technique of increasing the strength of the bone using injection instrument 1 is relatively high.

The bone to be strengthened using injection instrument 1 may be the femur, the vertebral body, a distal radius, or a proximal humerus of the target diagnosed with osteoporosis. With this, it is possible to prevent a fracture in the femur, the vertebral body, a distal radius, or a proximal humerus of the target diagnosed with osteoporosis.

The injection instrument and so forth according to the present invention have been described above on the basis of the embodiment, but the present invention is not limited to such embodiment. The scope of the present invention also includes an embodiment achieved by making various modifications to the embodiment that can be conceived by those skilled in the art and an embodiment configured by combining elements in different embodiments, without departing from the essence of the present invention.

### [Industrial Applicability]

The present invention is applicable for use as an injection instrument capable of increasing the strength of a bone.

### [Reference Signs List]

1, 1A injection device
5A, 5B inlet
10, 10A mixer
11, 12, 13, 14 portion
20 injector
21 side wall
22a, 22b, 22c, 22d, 22e side hole
24, 25 end portion
40 femur
41 cortical bone
42 cancellous bone
45 hole
46 graphics

## Claims

1. An injection instrument comprising:
an injector that is a cylindrical body having one or more holes,
wherein the injector receives a flow of a mixed solution in a state in which the injector is inserted in an opening formed in a bone of a target, the mixed solution being a mixture of a first solution and a second solution that hardens by being mixed with the first solution, and
the injector causes the mixed solution received to flow out of the injector from the one or more holes to permeate inside of the bone and harden.

2. The injection instrument according to claim 1,
wherein the one or more holes include:
one or more side holes located at one or more positions in a circumferential direction of the cylindrical body; or
one hole located at, among one end of the cylindrical body into which the mixed solution flows and an other end different from the one end, the other end.

3. The injection instrument according to claim 1,
wherein the one or more holes are a plurality of side holes located at a plurality of positions in an axial direction of the cylindrical body, and
a diameter of each of the plurality of side holes increases with increasing distance of the side hole from one end of two ends of the cylindrical body, the one end being an end into which the mixed solution flows.

4. The injection instrument according to claim 3,
wherein at least one of the plurality of side holes is oval in shape.

5. The injection instrument according to any one of claims 1 to 4,
wherein the opening is an opening formed by inserting the injector into the bone.

6. The injection instrument according to any one of claims 1 to 4,
wherein the opening is an opening formed by a drilling instrument different from the injector.

7. The injection instrument according to any one of claims 1 to 4, further comprising:
a mixer connected to one end of two ends of the injector, the one end being an end into which the mixed solution flows,
wherein the mixer receives the first solution and the second solution, and causes the mixed solution of the first solution and the second solution received to flow into the injector.

8. The injection instrument according to any one of claims 1 to 4,
wherein the target has a fracture in one femur, and the bone is an other femur of the target.

9. The injection instrument according to any one of claims 1 to 4,
wherein the bone is one of the following bones of the target diagnosed with osteoporosis: a femur; a vertebral body; a distal radius; and a proximal humerus.

10. The injection instrument according to any one of claims 1 to 4,
wherein the injector receives the flow of the mixed solution that has pregelatinized, and
the injector causes the mixed solution that has pregelatinized to flow out of the injector to permeate inside of the bone, gelatinize, and then harden.

11. A biomaterial comprising:
an injectable gel; and
a polymer of polymethyl methacrylate (PMMA) and a cross-linking agent.

12. The biomaterial according to claim 11,
wherein the injectable gel is a Tetra-Polyethyleneglycol (PEG) gel.
